# EUROPEAN PATENT SPECIFICATION

(11) **EP 0 715 393 B1**
(45) Date of publication and mention of the grant of the patent: **06.05.1999**
(21) Application number: 95830488.3
(22) Date of filing: 23.11.1995
(51) Int. Cl.: H02J 13/00, H05B 37/03

(54) **Widespread system for the detection of environmental pollution, in particular of atmospheric pollution**
Ausgebreitetes System zur Umweltverschmutzungsdetektion, insbesondere zur Luftverschmutzung
Système étendu pour la détection de pollution de l'environnement, en particulier la pollution atmosphérique

(30) Priority: 01.12.1994 IT RM940787
(43) Date of publication of application: 05.06.1996
(73) Proprietor: Rinaldi, Stefano, I-00198 Roma (IT)
(72) Inventor: Rinaldi, Stefano, I-00198 Roma (IT)
(74) Representative: Tonon, Gilberto

(56) References cited:
- EP-A- 0 082 080
- EP-A- 0 470 034
- CH-A- 683 136
- FR-A- 2 640 107
- FR-A- 2 668 674

## Description

The present invention relates to a distributed system for detection of environmental pollution, in particular for atmospheric pollution, with particular reference to urban areas.

More specifically, the present invention relates to a system of the type mentioned above that enables the parameters necessary for surveillance of the actual atmospheric and acoustic pollution situation in a metropolitan area to be made available in real time.

At present, environmental parameters are monitored by large control units positioned at the critical points in a city. As they have to detect, process and manage the data from the sensors on site, these control units are large in size and are generally installed on suitably equipped vans or in special container units. The high cost of each detection station means that the number of stations used has been limited. As a result of this, detection is also limited to a few points, which may be conditioned by strictly local phenomena and therefore may not give a correct and realistic analysis of the whole area.

The need has therefore been seen to develop an innovative product with a low cost and high expansion possibilities, in order to provide the authorities in charge with a fast, reliable and specific decisional instrument.

In the case of acoustic pollution also measurement of intensity is only representative within a limited area. The aim of the present invention is therefore to provide distributed monitoring of pollution (both acoustic and atmospheric), mainly generated by car traffic, with characteristics that can easily be integrated with and used alongside existing ones.

The system according to the present invention is based on small units containing only the parts relating to the sensors which, when connected to any public lighting point in the city, send data, using carrier current technology on the lighting network itself, to a single operations centre.

The extremely low cost of these small units and the ease with which they can be installed makes it possible for them to be widely distributed over the whole metropolitan area, thus allowing correct analysis of the whole territory in real time.

It is known from FR-A-2 668 674 to control and monitor the status of individual luminaires in a public lighting system using controllers in each luminaire which communicate with a central computor via the power distribution.

### Technical foundation of the invention

a) The system is widely distributed over the whole area of installation, detecting measurements at distances that are such as to avoid detriment to the reliability of data in particular conditions. This is generally the case when only a few control units are used, even if these are of a more sophisticated type. For example, in a large city detection can take place in thousands of different points instead of only a few points, generally less than ten. The more samples are taken at evenly distributed points, the more the results obtained correspond to the actual situation: positioning should be such as to form a network with meshes not exceeding 100 meters. It is important to note that, following installation of the system, which has a fixed one-off cost; the addition of new points for optimum environmental detection within the area involves extremely low costs.
b) The data is transmitted to a single centre in real time, thus allowing immediate processing to show critical areas, the influence of the rush-hour (traffic) and of the weather. There is also the option of examining, in a readable graphic form overlaid on the town plan, the value of the parameters measured in various areas, to identify critical points and if necessary intervene in real time to eliminate them; all this taking place within the operating centre and, if so desired, being made available to the public by means of a monitor.
c) The measurement points do not require personnel, thus eliminating the costs necessary for this. Furthermore, they have been specially designed for rapid and economic maintenance, the only operation taking place on site being replacement, which thus does not require specialised personnel. A special centre can be foreseen in which malfunctioning units can be repaired and made available to the service department again.
d) The system uses as its means of transmission the existing city lighting power distribution and node interconnection network, if necessary it can use the MV network (10 KV) by means of coupling systems for the frequency used or, if present, the line used to send the light-up command.
e) The system allows two-directional data transmission. This is of use for the lighting system Management, for example to enable partial and differentiated remote control of the light-up in each single light, as well as controls (light out of order).
f) Power supply to the electronic circuits of the elements in the system is taken from a trickle-charged battery charged by the voltage used for illumination during certain hours of the day, resulting in negligible power consumption.
g) The elements containing the sensors are housed in small weather-proof cases, and they are easily installed on the lamp posts themselves, on walls or hanging from the cables supplying the lighting elements.

According to the present invention a distributed system is provided for detection of environmental pollution, in particular atmospheric pollution, as defined in the enclosed claims.

The present invention will now be described with reference to a presently preferred embodiment thereof, given as a non-limiting example, and based on the enclosed drawings, in which:
figure 1 shows a block diagram of a detection unit;
figure 2 shows a block diagram of a unit as illustrated in figure 1;
figures 3a, 3b, 3c, 3d show block diagrams of analog detectors or sensors;
figures 4a and 4b show block diagrams of status detectors;
figures 5a, 5b and 5c show actuators;
figure 6 shows a wiring diagram for connection of sensor units and an MV/LV distribution station;
figure 7 shows a wiring diagram for connection between MV/LV station (concentrators) and Master, Co-ordinator and Communication Server units;
figure 8 shows a flow diagram for the data concentrator;
figures 9 and 10, taken together, show a flow diagram for the sensor unit;

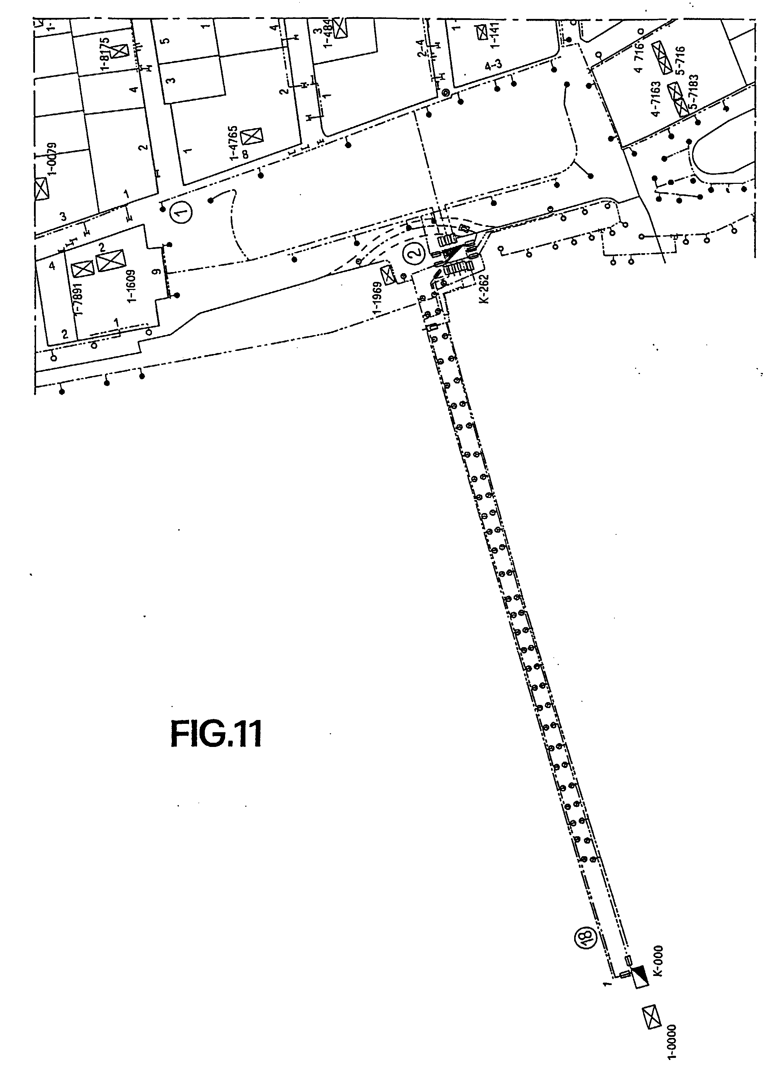

figure 11 shows a wiring plan to cover a city;

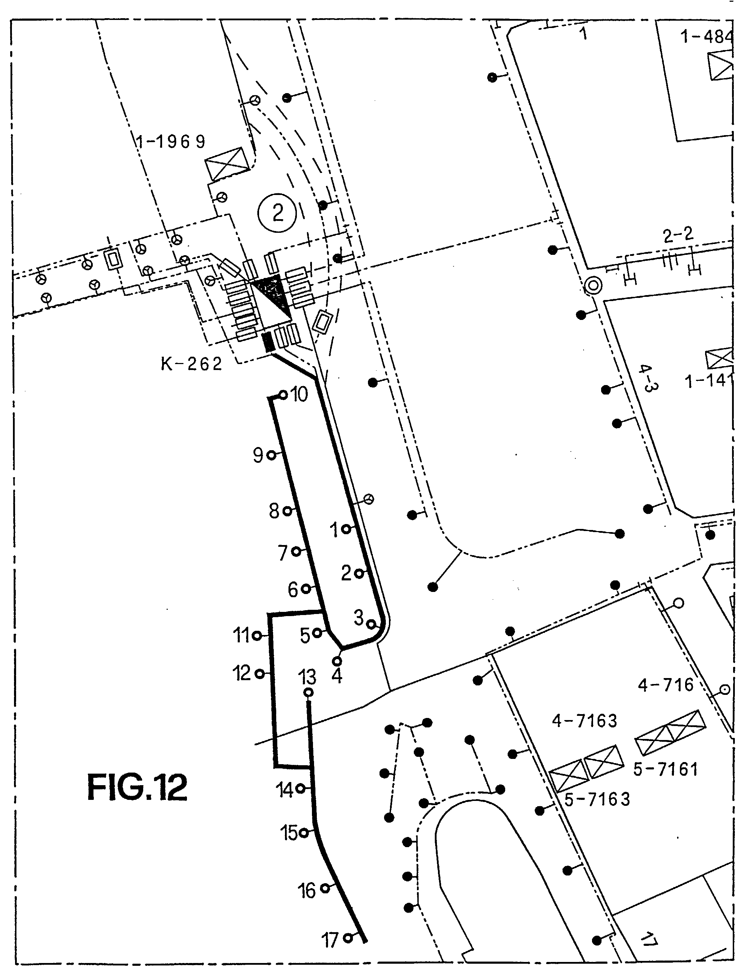

figure 12 shows a detail of the plan according to figure 11; and

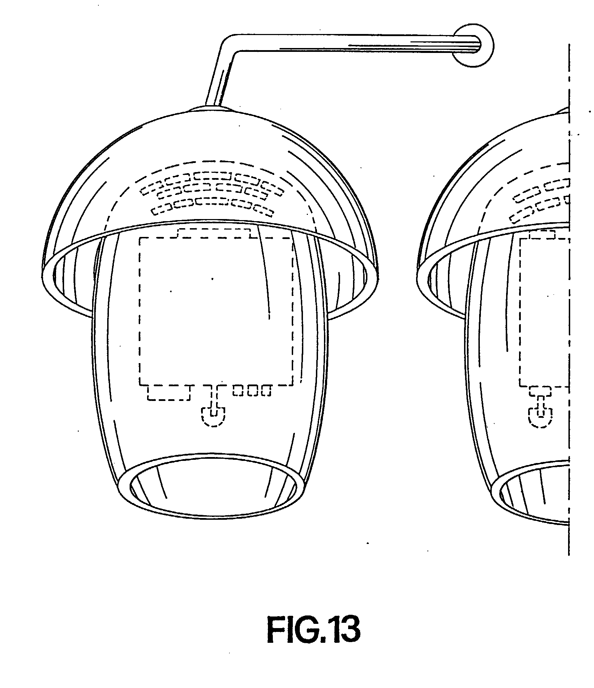

figure 13 shows a plan of a possible external structure for a sensor unit.

### DESCRIPTION

The system according to the invention is made up of a plurality of detection units connected directly to public lighting points; of a group of concentrators situated in correspondence with the MV/LV stations, and of Personal Computer type processors with the functions of Master, Co-ordinator and Communication Server situated in an operations centre.

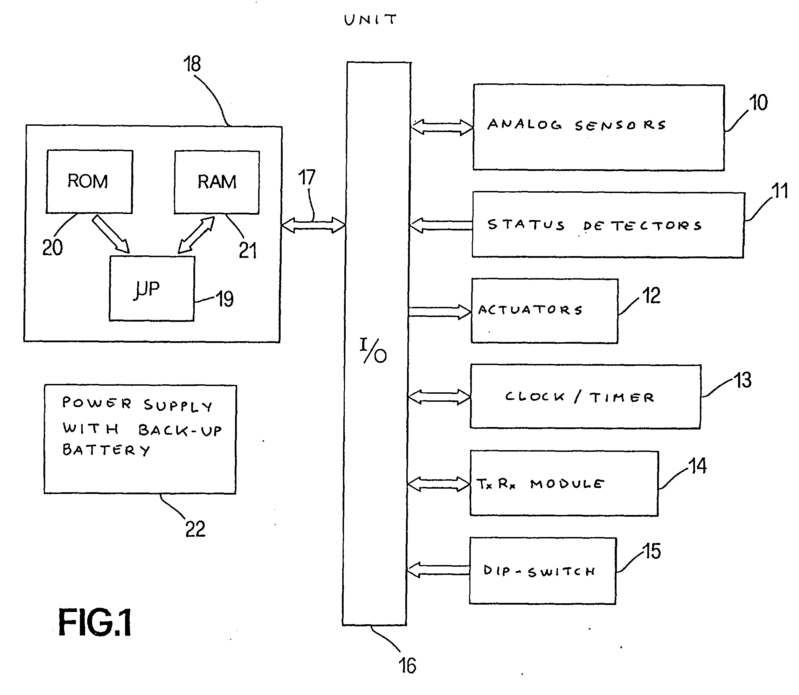

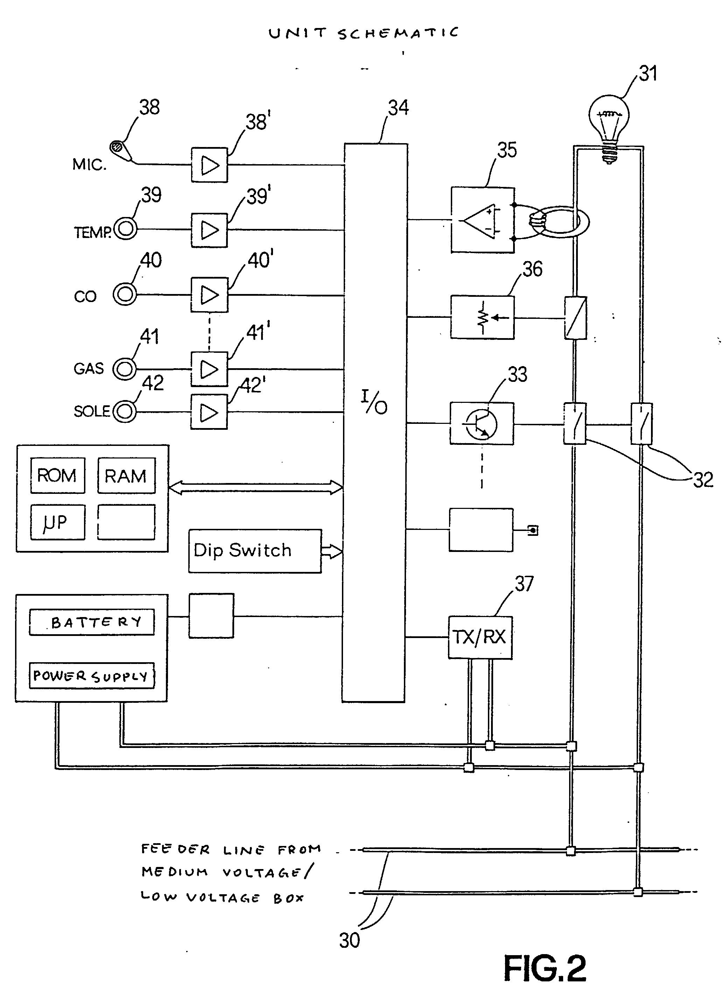

With reference now to figures 1 and 2, a detailed description will be given of the detection units directly connected to the public lighting points.

As shown in figure 1, a typical unit comprises a group 10 of analog sensors of the magnitude under measurement; a group 11 of status sensors, a group 12 of actuators, a clock/timer 13, a module 14 to transmit/receive signals to and from a remote station, and a group 15 of locally set hardware switches. The groups or function blocks 10-15 listed above are connected to an I/O interface 16 which communicates on a conventional bus 17 with a control unit 18, including a microprocessor unit 19 that co-operates with a resident program ROM 20 and a RAM 21 using a functional architecture well know to an expert in the field. The function blocks described above are powered by a local power supply 22 connected to the mains and are provided with a back-up battery.

In figure 2 the same elements indicated in figure 1 are shown, arranged in a specific circuit configuration.

With reference to figure 2, on a LV power supply line, indicated with 30, a lighting device 31 is connected, here illustrated as an example in the form of an incandescent lamp. The power supply to the lighting device 31 is controlled by switches 32 driven by a driver 33 connected to an I/O interface 34.

On the power supply line feeding the lighting device 31 is connected a current sensor of the "amperometric clip" type, indicated with 35 and connected to the interface 34. To the interface 34 are connected a partialization transformer 36, and a carrier current transceiver 37 to send and receive signals using conventional carrier current techniques on the electric power supply line 30 to and from a central unit, as will be more clearly illustrated in the following.

The following are connected to the I/O interface 34: the sensors mentioned above, and in particular a microphone 38 to detect the environmental noise level, a temperature sensor 39, a carbon monoxide sensor 40, a gas sensor 41 (optionally sub-divided into a plurality of specific sensors for gases such as NOₓ, SO₂, SO₃, hydrocarbons, etc..), and a solar ray sensor 42.

These sensors, the number of which may vary according to the number of environmental parameters to be kept under control, are connected to signal normalisation and analog-to-digital conversion units, indicated using the same reference numbers given for the sensors, primed (`).

The rest of figure 2 has already been illustrated with reference to the description of figure 1 above.

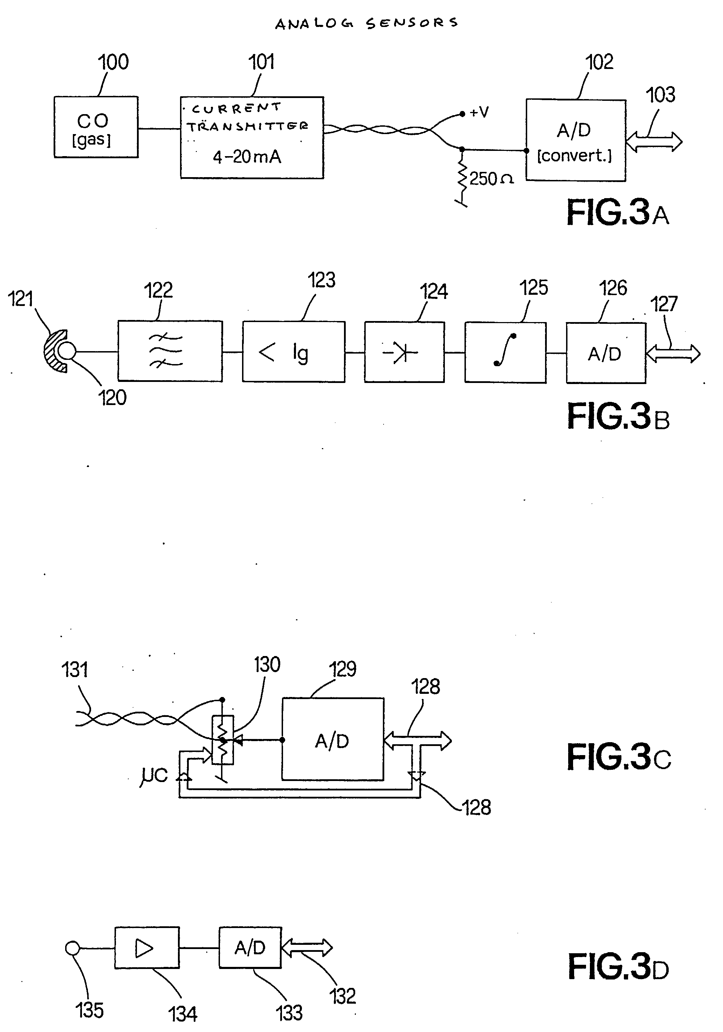

With reference to figures 3a, 3b, 3c, 3d, a detailed illustration of the block diagrams of analog type sensors will now be given.

The type illustrated in figure 3a is typically destined for detection of gas. This sensor comprises a unit 100, for example suitable for detection of carbon monoxide, and which in commonly used sensors is of a resistive type, so that by means of a current/voltage transducer 101a voltage is supplied which, when applied to a conventional analog-to-digital converter 102, provides on a bus 103 data in a digital form applied to the I/O unit described above.

Figure 3b shows an environmental noise sensor. A microphone capsule 120 with line output as a function of the noise pressure and equipped with a conventional wind guard 121 is connected to a pass band filter 122. The transfer characteristics of the pass band filter 122 will be selected according to the particular type of noise or sound whose level is to be evaluated. The output from the filter 122 is conducted to a logarithmic amplifier 123 for conversion of the signal, linearly proportional to the noise, into logarithmic units (dB). A rectifier 124 and an integrator 125 with an appropriate time constant provide a normalised output conducted to an analog-to-digital converter 126 connected to a bus 127 connected as indicated above with the I/O unit.

Figure 3c shows the circuit arrangement in blocks for those sensors requiring automatic calibration according to specific parameters supplied by an applications program.

In this case the bus 128 connected to the I/O unit is two-directional and, on one side (128A) allows transit of digital signals coming from an analog-to-digital converter 129, while on the other side (128B) it allows a controlled member 130, for example for variable conditioning or adaptation of an output 131 of a sensor, to be varied using digital data. A typical example of controlled member might be a digitally controlled potentiometer (E2POT) or a stepper motor, or the like.

Figure 3d shows a generic arrangement of the "uncommitted" type, destined for particular uses, in which the signal conditioned by a normalizer amplifier 134 arrives at the bus 132, connected to an analog-to-digital converter 133. The input 135 from the normalizer amplifier 134 might be an electric signal that is a function of the environmental temperature, humidity, quantity and distribution of solar irradiation, which is important for evaluation of photochemical smog, or the like.

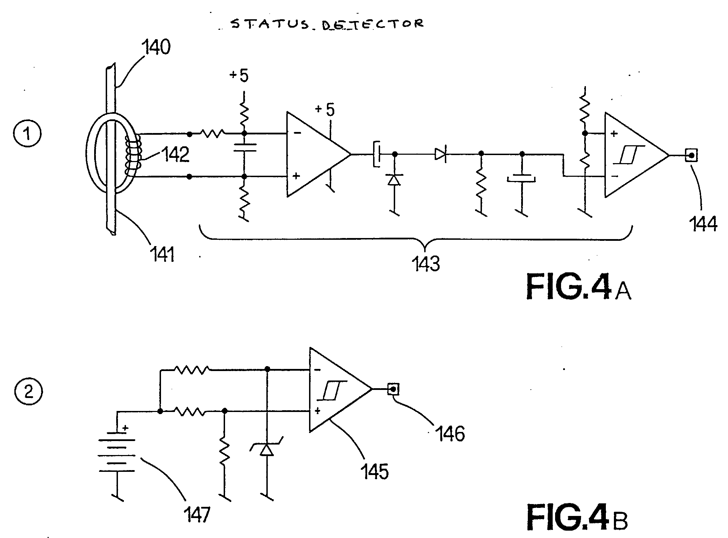

Figures 4a and 4b show two status detectors for operating devices having an ON/OFF condition only, and thus a single bit digital output.

Figure 4a shows a generic electrical cable 140 connected to a conventional amperometric sensor with magnetic core 141, the secondary 142 of which is connected to a conventional group 143 comprising an amplifier, a rectifier and a Schmitt threshold circuit that supplies a signal indicating the presence or absence of current flowing on the cable 140 on a terminal 144 (for instance to indicate deactivation of a public lighting element: lamp)

Figure 4b shows a comparator 145 arranged, for example, to provide information on the conditions of a back-up battery 147 on a terminal 146.

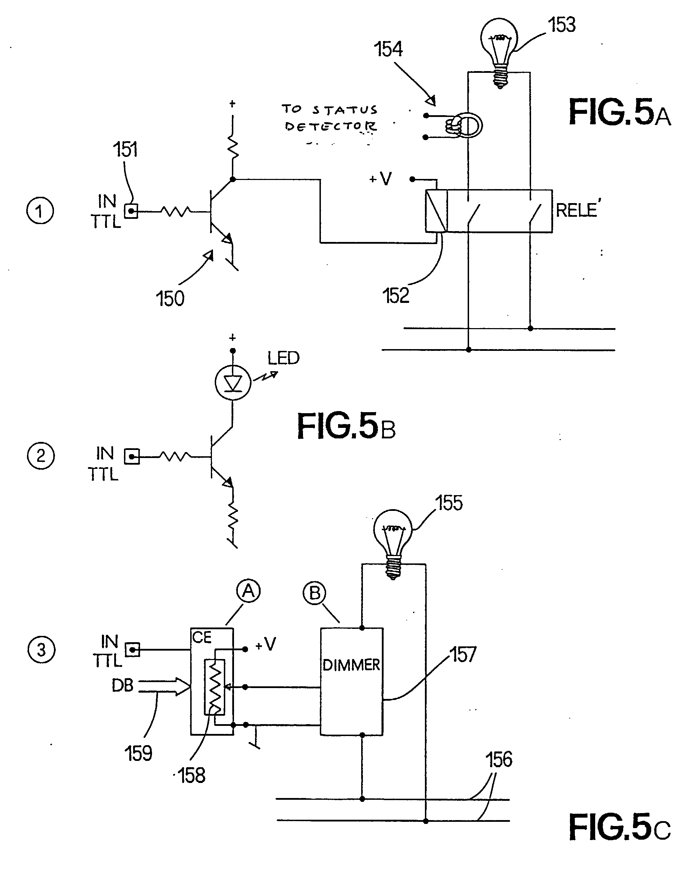

Figures 5a, 5b, 5c show typical examples of actuators.

Figure 5a shows an actuator comprising a power translator 150 which, starting from a logic level command on the terminal 151, drives a pair of relays 152 to control light-up of a lamp 153. In the lamp cables 153 a status detector of the type illustrated in figure 4a can be installed, indicated with 154.

Figure 5b shows a generic status detector for one dimension, such as the status of a floating battery.

Figure 5c shows a power variation actuator in which the power delivered to a load 155 by a feeder 156 is adjusted by means of a TRIAC variator 157 controlled by a variable resistor 158 according to a value pre-set on a digital bus 159.

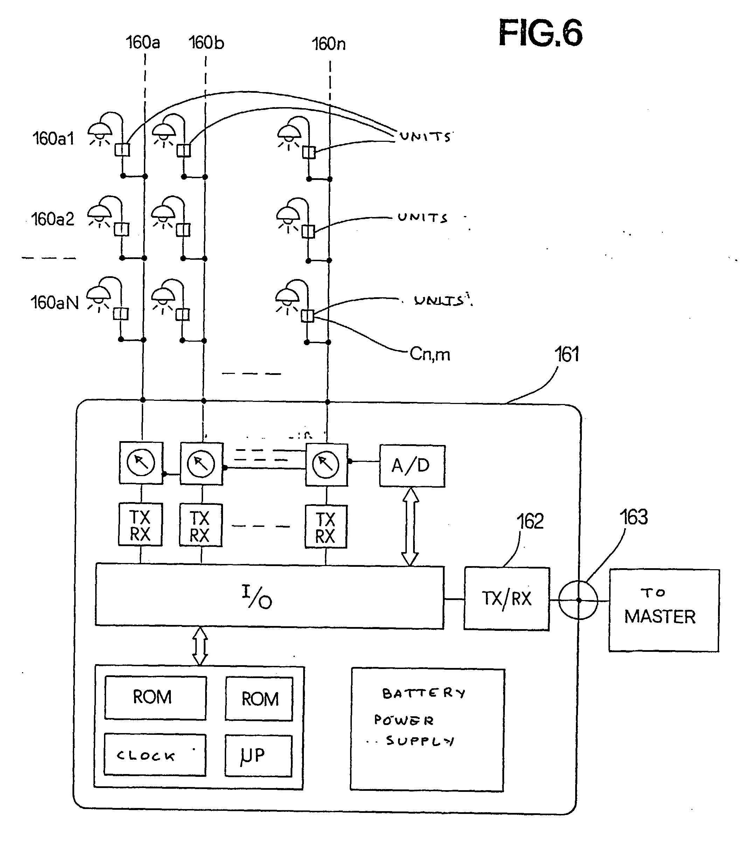

Figure 6 shows a summary of the wiring of a plurality of sensor units, local processor and master management unit.

There are a plurality of unit groups 160a, 160b, ....160n, each of which comprises a plurality of subgroups 101a1, 101a2,... 101aN, to each of which is connected a group of sensors/actuators or cells such as those described above, indicated with Cn, m. The subgroups or columns 160a, ... 160n are connected to a local unit 161 described above, the description of which will not be repeated for the purpose of brevity, and which comprises a carried wave transceiver 162 arranged to be operatively connected at 163 (that is to say by a physical support made up of the electric power distribution cables) with a master unit according to one of the configurations well known from the state of the art for data distribution networks (star, ring, and variations and combinations thereof).

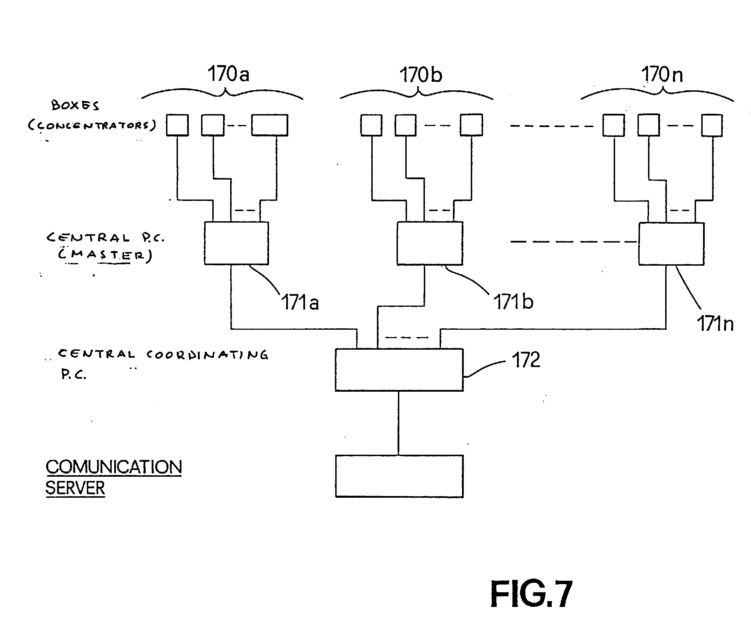

Figure 7 shows a wiring diagram for connection of the MV/LV station (concentrators) and the Master, Co-ordinator and Communication Server units.

As illustrated, 170a, 170b, ... 170n are the station units or concentrators shown in block form. These units 170a, ... 170n are connected in the central station to Master processors 171a, 171b, ... 171n, which in turn are connected to a co-ordinator processor in the central station 172, which in turn is connected to a communication server 173 using architecture well known in the field and which will therefore not be described in further detail.

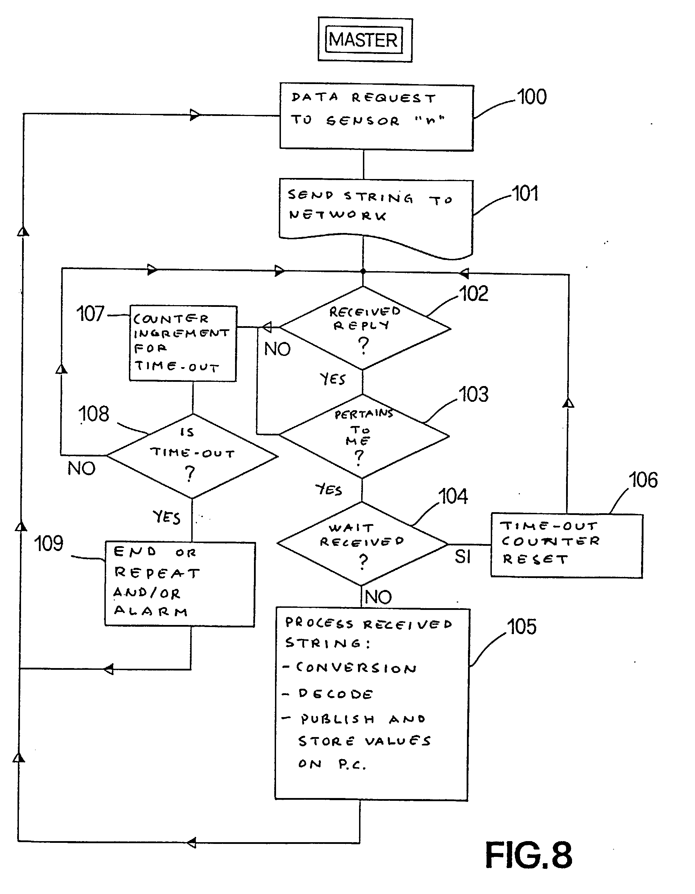

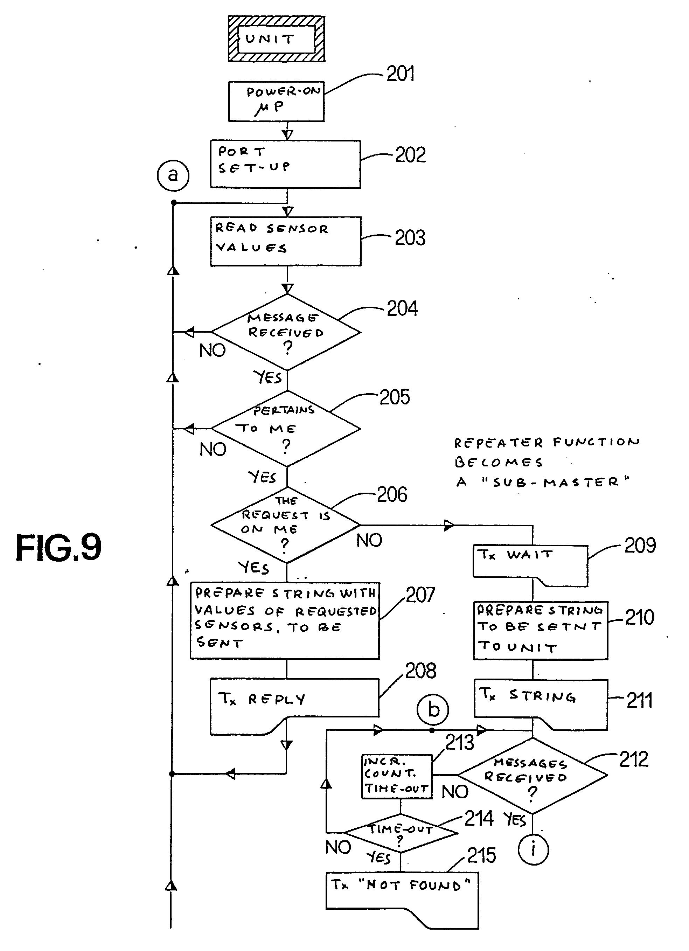

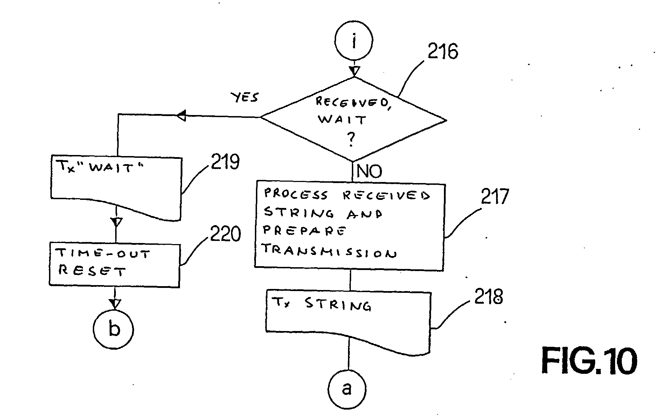

For the sake of completeness, the flow diagram for the software for the data concentrator and for the unit or group of sensors, with reference to figures 8 and 9, 10, respectively.

### CONCENTRATOR

### 1100 Data requested from Unit n

The Concentrator performs a polling operation to learn the data for each Unit for which it is responsible, for which it periodically prepares a string to send to the network with unit n as its destination code.

### 1101 String Sent to network

The string prepared is transmitted on the network

### 1102 Reply received?

The Concentrator reverts to receive state and zeros the time-out timer. If no message has been received, then 1107, otherwise 1103

### 1103 Is the message for me?

A great many messages pass along the network. Each string contains the destination of the message it contains. If the message is not addressed to the concentrator, then 1107, otherwise 1104

### 1104 Was the message wait?

The message might be for me, but it might not give me a reply to my request, as the unit interrogated cannot be reached directly but only through another relay unit. In this case the relay unit emits a **wait** message (see 1209 in the Unit flow diagram). If the message is wait, then 1106, otherwise 1105.

### 1105 Process string received

The Concentrator has received the string containing the status of the Unit questioned. This string is processed, decoded and stored in a matrix. This matrix, which contains all the values for all the Units for which the Concentrator is responsible, will then be sent, upon request, to the Master.

### 1106 Reset time-out timer.

If the wait message has been received, then zero the time-out timer and return to 1102 to await receipt.

### 1107 Increase time-out timer

Each time a message is not received the time-out timer is increased, so as to know how long it is since a message was received.

### 1108 Time-out?

The value of the time-out timer is compared with a pre-set value equivalent to the maximum waiting time. If this time has passed, then 1109, otherwise 1102.

### 1109 End or repeat and/or alarm

If within a certain amount of time no reply has been received from the unit, try again by sending another request (1101). If, even after this further attempt, no reply is received then the unit is set down as inoperative. When the Concentrator dialogues with the Master it will inform the latter of this malfunction.

### SENSOR/ACTUATOR UNITS

### 1201 Power-on procedures

When the power is turned on the Unit has to carry out a series of procedures necessary to initialise and check the correct operation of all its components. For example, gas sensors need to be switched on for approximately 10 minutes before stabilising.

### 1202 Gate configuration

A special initialisation procedure is the one for configuration of the I/O gates, which varies according to the number of sensors/actuators installed in the Unit itself.

### 1203 Read sensor values

The microcontroller reads the state of all its sensors, with a frequency of 1 second, then performs the necessary conversions and calculations so as to update its internal data matrix.

### 1204 Any message received?

The Unit is always in receive mode, awaiting interrogation by the Concentrator. If no message has been received, then 1203, otherwise 1205.

### 1205 Is the message for me?

A great many messages pass along the network. Each string contains the destination of the message it contains. If the message is not addressed to the concentrator, then 1203, otherwise 1206.

### 1206 Is the message through me?

Each Unit can also become a Relay-unit. The sender of the message (Concentrator or other Relay-unit) always indicates the final destination of the message. If the Unit in question is also a relay unit and the final destination is another unit dependent on it, then 1209, otherwise 1207.

### 1207 Prepare the reply string.

If I am the final destination of the message, take the necessary data from the matrix and prepare the string to be sent.

### 1208 Tx reply.

The string prepared as indicated is transmitted over the network.
From 1209 onwards the Unit is acting as a relay station.

### 1209 Tx wait.

The Unit has become a relay station. It sends the wait message to its Concentrator or to another Relay unit.

### 1210 Prepare String to send to Unit.

It prepares the string to be sent in the same way that the Concentrator does.

### 1211 Tx String.

The String prepared as indicated is transmitted over the network.

### 1212 Reply received?

Like the Concentrator, the relay station also carries out the same receive cycle (from 1102 to 1109). If a reply is received, then 1216, otherwise 1213.

### 1213 Increase time-out timer

Equivalent to 1107.

### 1214 Time-out?

Equivalent to 1108. If not, go to 1212, otherwise go to 1215.

### 1215 Tx Not Found

Partly equivalent to 1109. If no reply has been received from the Unit called, I inform my Concentrator, or the Relay Unit that requested the information, that the unit in question is not answering.

### 1216 Was the message wait?

Equivalent to 1104. If the message is wait, then 1219, otherwise 1217.

### 1217 Process string received

Equivalent to 1105. The string received is processed and prepared for Re-transmission.

### 1218 Tx string.

The string prepared as indicated is sent over the network. Continue at 1203.

### 1219 Tx Wait.

Equivalent to 1209. Sends the wait message to its Concentrator of the other Relay Unit, as the Unit interrogated is in turn a relay station. The process is obviously an iterative one. Continue from 1212.

The present invention has been described with reference to a currently preferred embodiment thereof, but it will be understood that in practice it is possible for a person skilled in the art to make alterations and adjustments without departing from the scope of the present industrial property rights.

## Claims

1. A distributed system for detection of environmental pollution, in particular of atmospheric pollution, characterised in that it comprises, joined to a distributed system of lighting points (31) within an urban area, a plurality of detection units comprising sensors (38, 39, 40, 41, 42) for environmental pollution parameters, said plurality representing a total number lower than that of said lighting points; carrier current communication means (14, 37, 162) between a central management unit and the individual detection units, said carrier current communication means being arranged so as to use the lines (30) for transport and distribution of electric power to said lighting points or, a dedicated control line commanding activation of said lighting points if one is present, said detection units being provided with a microprocessor unit 18 operatively associated with said environmental pollution parameter sensors and a carrier current transceiver (14, 37, 162).

2. A distributed detection system according to claim 1, characterised in that said detection units are self-supplied by means of a bank of trickle-charged batteries.

3. A distributed detection system according to claims 1 or 2, characterised in that said detection units in addition to said environmental pollution parameter sensors are provided with sensor means for detection of operating parameters and possible malfunctions in one or more lighting points.

4. A distributed detection system according to claim 3, characterised in that said detection units are further provided with command means for said lighting points.

5. A distributed detection system according to one or more of the preceding claims, characterised in that said detection units operatively associated with a carrier current transceiver are arranged so as also to operate as communications relay stations.

6. A distributed detection system according to one or more of the preceding claims, characterised in that the environmental pollution parameter sensors are connected to said microprocessor units by means of analog-to-digital converters, with the optional interposition of devices for the conditioning and pre-professing of the signals supplied by said environmental pollution parameter sensors.

7. A distributed detection system according to one or more of the preceding claims, characterised in that said detection units are made using modular technology.

8. A distributed detection system according to one or more of the preceding claims, characterised in that said detection units are made as self-contained units installed in correspondence with the lighting points in an urban area.

## Patentansprüche

1. Verteiltes System zur Umweltverschmutzungserfassung, insbesondere zur Luftverschmutzungserfassung,
**dadurch gekennzeichnet, daß**
das Erfassungssystem in Verbindung mit einem verteilten System von Beleuchtungsstellen (31) innerhalb eines städtischen Gebiets eine Vielzahl von Erfassungseinheiten, die Sensoren (38, 39, 40, 41, 42) für Umweltverschmutzungsparameter aufweisen, wobei die Anzahl der Sensoren geringer ist als die der Beleuchtungsstellen, und Trägerfrequenz-Kommunikationseinrichtungen (14, 37, 162) zwischen einer zentralen Verwaltungseinheit und den einzelnen Erfassungseinheiten umfaßt, wobei die Trägerfrequenz-Kommunikationseinrichtungen eingerichtet sind, Leitungen (30) zur Übertragung und Verteilung von elektrischer Energie zu den Beleuchtungsstellen oder, sofern vorhanden, eine bestimmte Steuerungsleitung zur Anweisung der Aktivierung der Beleuchtungsstellen zu verwenden, und wobei die Erfassungseinheiten mit einer Mikroprozessoreinheit 18 ausgestaltet sind, die mit den Umweltverschmutzungsparametersensoren und einer Trägerfrequenz-Übertragungseinrichtung (14, 37, 162) in Betriebsverbindung stehen.

2. Verteiltes Erfassungssystem nach Anspruch 1, **dadurch gekennzeichnet, daß** die Erfassungseinheiten eine Eigenenergieversorgung in Form eines Satzes Erhaltungsladungsbatterien aufweisen.

3. Verteiltes Erfassungssystem nach Anspruch 1 oder 2, **dadurch gekennzeichnet, daß** die Erfassungseinheiten zusätzlich zu den Umweltverschmutzungsparametersensoren mit Sensoreinrichtungen zur Erfassung von Betriebsparametern und möglichen Fehlfunktionen bei einer oder mehreren Beleuchtungsstellen versehen sind.

4. Verteiltes Erfassungssystem nach Anspruch 3, **dadurch gekennzeichnet, daß** die Erfassungseinheiten ferner mit Befehlseinrichtungen für die Beleuchtungsstellen versehen sind.

5. Verteiltes Erfassungssystem nach zumindest einem der vorhergehenden Ansprüche, **dadurch gekennzeichnet, daß** die mit einer Trägerfrequenz-Übertragungseinrichtung in Betriebdverbindung stehenden Erfassungseinheiten ebenso als Kommunikationsrelaisstationen betreibbar sind.

6. Verteiltes Erfassungssystem nach zumindest einem der vorhergehenden Ansprüche, **dadurch gekennzeichnet, daß** die Umweltverschmutzungsparametersensoren mit den Mikroprozessoreinheiten mittels Analog-Digital-Umwandlungseinrichtungen verbunden sind, wobei wahlweise Vorrichtungen zur Gestaltung und Vorverarbeitung der durch die Umweltverschmutzungsparametersensoren zugeführten Signale zwischenschaltbar sind.

7. Verteiltes Erfassungssystem nach zumindest einem der vorhergehenden Ansprüche, **dadurch gekennzeichnet, daß** die Erfassungseinheiten unter Verwendung modularer Verfahren hergestellt sind.

8. Verteiltes Erfassungssystem nach zumindest einem der vorhergehenden Ansprüche, **dadurch gekennzeichnet, daß** die Erfassungseinheiten als in sich geschlossene Einheiten hergestellt sind, die entsprechend den Beleuchtungsstellen in einem städtischen Gebiet angebracht sind.

## Revendications

1. Système distribué pour la détection de la pollution de l'environnement, en particulier de la pollution atmosphérique, caractérisé en ce qu'il comprend, associé à un système distribué de points d'éclairage (31) dans une zone urbaine, une pluralité d'unités de détection comprenant des capteurs (38, 39, 40, 41, 42) pour des paramètres de pollution d'environnement, ladite pluralité représentant un nombre total inférieur à celui desdits points d'éclairage, un moyen de communication de courant porteur (14, 37, 162) entre une unité de gestion centrale et les unités de détection individuelles, ledit moyen de communication de courant porteur étant disposé afin d'utiliser les lignes (30) pour le transport et la distribution de l'énergie électrique auxdits points d'éclairage ou, une ligne de commande dédiée commandant l'activation desdits points d'éclairage si un est présent, lesdites unités de détection étant pourvues d'une unité de microprocesseur (18) associée fonctionnellement auxdits capteurs de paramètre de pollution d'environnement et a un émetteur-récepteur de courant de porteur (14, 37, 162).

2. Système distribué de détection selon la revendication 1, caractérisé en ce que lesdites unités de détection sont auto-alimentées au moyen d'une banque de batteries chargées lentement.

3. Système distribué de détection selon les revendications 1 ou 2, caractérisé en ce que lesdites unités de détection en plus desdits capteurs de paramètre de pollution d'environnement sont pourvus d'un moyen de capteur pour la détection de paramètre de fonctionnement et de dysfonctionnements possibles dans un ou plusieurs points d'éclairage.

4. Système distribué de détection selon la revendication 3, caractérisé en ce que lesdites unités de détection sont en outre pourvues d'un moyen de commande pour lesdits points d'éclairage.

5. Système distribué de détection selon l'une ou plusieurs des revendications précédentes, caractérisé en ce que lesdites unités de détection associées fonctionnellement à un émetteur-récepteur de courant porteur sont disposées afin de fonctionner aussi comme des stations de relais de communications.

6. Système distribué de détection selon l'une ou plusieurs des revendications précédentes caractérisé en ce que les capteurs de paramètre de pollution d'environnement sont raccordés auxdites unités de microprocesseur au moyen de convertisseurs analogiques numériques, avec l'interposition optionnelle de dispositifs pour le conditionnement et le prétraitement des signaux fournis par lesdits capteurs de paramètre de pollution d'environnement.

7. Système distribué de détection selon l'une ou plusieurs de revendications précédentes, caractérisé en ce que lesdites unités de détection sont fabriquées en utilisant une technologie modulaire.

8. Système distribué de détection selon une ou plusieurs des revendications précédentes, caractérisé en ce que lesdites unités de détection sont fabriquées comme des unités contenues installées en correspondance avec les points d'éclairage dans une zone urbaine.
